# EUROPEAN PATENT SPECIFICATION

(11) **EP 1 490 008 B1**
(45) Date of publication and mention of the grant of the patent: **03.06.2009**
(21) Application number: 03722380.7
(22) Date of filing: 01.04.2003
(51) Int. Cl.: A61F 15/00, A61F 13/15

(54) **PACKAGING MATERIAL SHEET WITH AT LEAST ONE LINE OF WEAKNESS, AND PACKAGING MATERIAL**
VERPACKUNGSMATERIALBAHN MIT WENIGSTENS EINER SCHWÄCHUNGSLINIE UND VERPACKUNGSMATERIAL
FEUILLE DE MATERIAU D'EMBALLAGE COMPORTANT AU MOINS UNE LIGNE DE RUPTURE, ET MATERIAU D'EMBALLAGE ASSOCIE

(30) Priority: 02.04.2002 DE 10214579
(43) Date of publication of application: 29.12.2004
(73) Proprietor: Johnson & Johnson GmbH, 41470 Neuss (DE)
(72) Inventor: SCHOELLING, Hans-Werner, 58286 Ennepetal (DE); JANSSEN, William, 58332 Schwelm (DE); HULL, Raymond, J., Hampton, NJ 08876 (US)
(74) Representative: Metten, Karl-Heinz
(86) International application number: PCT/EP2003/003396
(87) International publication number: WO 2003/082174

(56) References cited:
- EP-A- 0 597 446
- EP-A- 0 988 847
- EP-A- 1 297 805
- CH-A- 340 763
- DE-U- 20 115 829
- GB-A- 758 879
- US-A- 4 170 305

## Description

The invention relates to a tampon wrapped in a packaging material sheet which has at least one line of weakness in order to open a pack produced from the packaging material for a product, in particular a mass product.

Such a packaging material is disclosed by EP 0 597 446 Al. The line of weakness described is a line which runs through the packaging material and is composed of a row of weak points of different size and type, so that the material comprises two parts connected by the line of weakness. The line of weakness can also be composed of a row of weak points which have a particular geometry, for example arrows or semicircles.

The packaging material disclosed in EP 0 597 446 A1 is mainly used to pack tampons. In order to open the tampon pack or other packs, the two parts of the packaging material separated from each other by the line of weakness are rotated in relation to each other or at least displaced in the opposite directions, so that the line of weakness is destroyed and two pack parts completely separated from each other remain.

These pack parts enclose the product substantially completely, even after the destruction of the line of weakness, and are pulled off from the packed product in opposite directions in a further step.

In particular in the case of products such as tampons for feminine hygiene, which are enclosed very closely by the packaging material for space-saving reasons and to avoid material damage, the problem arises that. the individual pack parts are difficult to pull off the product or can no longer be pulled off the product, particularly if the product expands. This may be the case primarily in the aforementioned tampon for feminine hygiene, but also in the case of other products made of compressed materials, since these can continue to expand after manufacture and after packaging.

It is accordingly an object of the present invention to provide an improved packed tampon for feminine hygiene, which permits the product to be unpacked simply, above all tightly packed products which can still expand after being packed.

This object is achieved by a tampon wrapped in a packaging material as claimed in claim 1. Claims 2 to 18 identify particularly advantageous embodiments of the tampon according to the invention.

After the destruction of the at least one line of weakness, a packaging material sheet with at least one line of weakness can be peeled off theproduct."Can be peeled off" is to be understood here to mean that the user can grip the packaging material in at least one sub-area after the at least partial destruction of the line of weakness and can pull off or remove the packaging material sheet completely from the product. The packaging material sheet ensures that the three-dimensional structure of the packaging material sheet is completely or gradually broken down with the destruction of the line of weakness. The packaging material sheet can be peeled off the product without any stresses which may occur as a result of expansion of the product counteracting the unpacking of the product, as is the case in the prior art described above.

The packaging material sheet can preferably be peeled off the product in one piece. This has the advantage that the user or customer, after opening the product, only holds and must dispose of one packaging part. This is significant in particular in the case of cellophane sleeves, which are normally used for packing tampons for feminine hygiene, since a plurality of individual cellophane parts are difficult to handle, because of their frequent electrostatic charge. However, the problem also applies to other packaging materials.

In a preferred embodiment of the packaging material sheet, the at least one line of weakness has at least one end point, preferably at least two end points arranged at a distance from each other, within the packaging material sheet. This ensures in particular that the packaging material sheet can be peeled off in one piece. Furthermore, controlled peeling of the packaging material sheet is possible. In a special embodiment, the end points can also be reinforced, in order to prevent the packaging material sheet tearing beyond the end points.

The at least one line of weakness can advantageously be destroyed by means of compression and/or bending or by means of the at least partially opposed action of force on the packaging material sheet. For the user, this permits the rapid and simple destruction of the line of weakness and the opening of the product. Additional tear-off strips or overlapping parts of the packaging material sheet are not needed. The stability of the line of weakness is in this case matched to the product to be packed, primarily to its flexibility, in order that the user does not have to expend any great force to destroy the line of weakness, on the one hand, but on the other hand inadvertent destruction of the line of weakness is avoided.

In this connection, it should be noted that, as opposed to the packaging material described in EP 0 597 446A1, slight expansion of the product, in the case of the packaging material sheet can even facilitate unpacking. If the line of weakness is destroyed, and if the product was prestressed by subsequent expansion against the packaging material sheet, then the packaging material sheet will generally lift off the product easily at a point, so that it can be gripped without difficulty by the user and, as described above, can be peeled off.

The at least one line of weakness is preferably curved and/or it runs at least partially in at least two directions differing from each other. As a result, following the destruction of the line of weakness, the product can be unpacked more easily or the packaging material sheet can be peeled off more easily, since any prestresses which may be present are already relieved in a plurality of directions. Furthermore, such a line of weakness or the interaction of a number of lines of weakness lead to one end of the pack being available to be gripped by the user in order to peel off the packaging material sheet, after the at least partial destruction of the at least one line of weakness.

The at least one line of weakness preferably has a U-shaped or a sinusoidal course. According to a further embodiment, two lines of weakness arranged at a distance from each other are provided with a curved course or there are two mutually crossing lines of weakness, the mutually crossing lines of weakness preferably running substantially in straight lines. By choosing the course of the line of weakness, the unpacking or unpeeling actions can be made substantially easier, depending on the product to be packed. It has transpired that, in particular when packing cylindrical products, such as tampons for feminine hygiene, rapid and simple removal of the product from the pack can be ensured by a course of the at least one line of weakness as described above.

The at least one line of weakness preferably comprises weak points arranged at a distance from one another. The weak points can have different and/or varying dimensions. As a result, if the weakening of the line of weakness is adequate for unpacking the product, the overall stability of the packaging material sheet is not reduced, so that inadvertent damage or destruction of the packaging material sheet or its inadvertent opening is avoided.

The lines of weakness or the weak points can be formed by means of perforation and/or a reduced material thickness in the area of the line of weakness. Forming the line of weakness only by means of a reduced material thickness of the packaging material sheet has the advantage that the packed product is protected against dampness and contamination from the outside without further measures.

In this respect it is possible to utilize a packaging material which comprises a web of packaging material sheets, as described above, adjoining one another. The individual packaging material sheets are designed such that they can be separated from the web in order to pack a product. For mass production, in particular, such packaging material sheets cohering in a web have the advantage that they can be supplied quickly and without difficulty to the corresponding packaging device. The packaging material web is normally wound up on itself, so that a roll of the packaging material sheets lined up in a row is made available.

In addition, the web preferably has a line of weakness between the individual packaging material sheets, for example a reduced material thickness and/or a perforation. As a result, the individual material sheets for packaging are simple to separate from the web and to process. The packaging material sheet can be provided with at least one line of destruction for opening a pack produced from the packaging material for a product, the at least one line of destruction extending over at least part of the longitudinal extent of the pack after the product has been packed. This achieves the situation where the parts of the pack which are produced following the destruction of the line of destruction and which are substantially sleeve-like, do not extend completely around the circumference of the product to be packed, at least over part of their longitudinal extent. Since, in this way, the friction between the sleeve-like parts of the pack which are produced and the product to be packed is reduced, the sleeve-like parts can be pulled off the packed product more easily, substantially in the longitudinal direction of said product.

In one embodiment, the line of destruction, following the packing of the product, extends over 20% of the longitudinal extent of the product, preferably over 50% of the length of the packed product and particularly preferably over70% of the length of the packed product.

The line of destruction may be a line of weakness as has already been described in connection with the tampon of the present invention.

The line of destruction is preferably curved or sinusoidal, it being possible for it to be formed around the circumference of the packed product with one curve or a plurality of curves.

The invention will be described in detail below using the appended schematic drawings of a number of exemplary embodiments, in which:
Fig. 1 shows a web with packaging material sheets according to a first embodiment of the invention;
Figs 2a and 2b show a tampon which has been packed with a packaging material sheet according to Fig. 1, and also individual unpacking steps;
Fig. 3 shows a web with packaging material sheets according to a second embodiment of the invention;
Figs 4a, 4b, 4c and 4d show a tampon which has been packed with the packaging material sheet shown in Fig. 3, and also individual unpacking steps;
Fig. 5 shows a web with packaging material sheets according to a third embodiment of the invention;
Figs 6a, 6b, 6c and 6d show a tampon which has been packed with the packaging material sheet shown in Fig. 5, and also individual unpacking steps;
Figs 7a and 7b show two tampons packed with a fourth embodiment of the packaging material sheet, in two different variants;
Fig. 8 shows a tampon packed with a fifth embodiment of the packaging material sheet according to the invention;
Figs 9a, 9b show a first embodiment of a packaging material sheet according to the invention, and also a tampon which has been packed with such a packaging material sheet; and
Figs10a, 10b show a second embodiment of a packaging material sheet according to the invention, and also a tampon which has been packed with such a packaging material sheet.

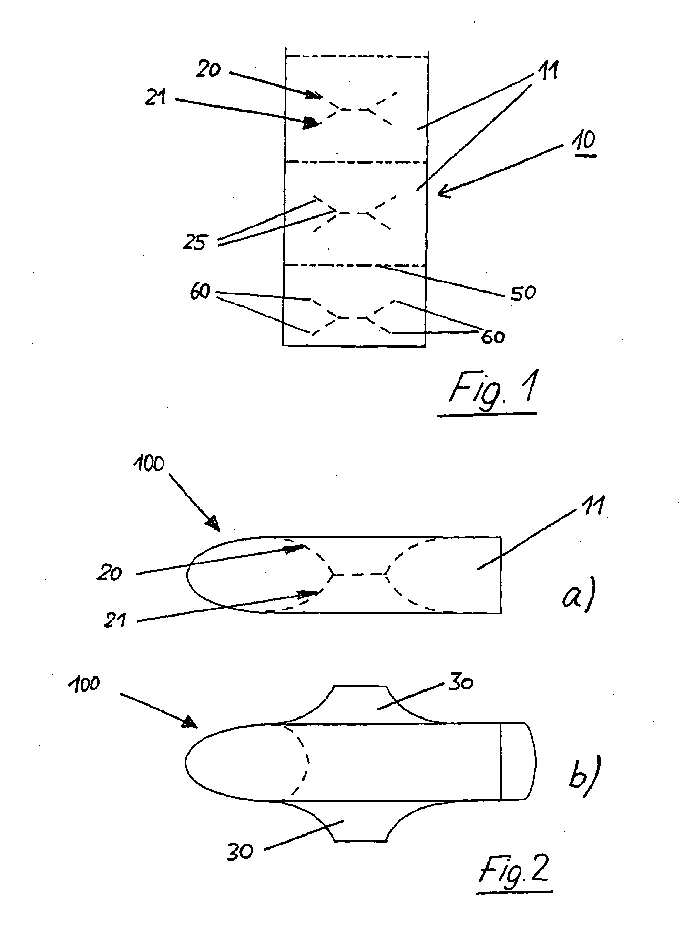

Fig. 1 shows a cellophane web 10 from which individual sheets 11 connected to one another via a line of weakness 50 can be separated and are used as packaging material sheets 11 for a product, for example a tampon. The packaging sheet 10 is essentially rectangular and, in its central area, is provided with two mutually crossing lines of weakness 20, 21, which run together at the center to form a single line of weakness.

The lines of weakness 20, 21 consist of individual weakening sections or weak points 25, in which the packaging material has a reduced thickness, and end in a total of four end points 60.

Fig. 2a shows a tampon 100 for feminine hygiene packed in the packaging material sheet 11 shown in Fig. 1. The lines of weakness 20, 21 are arranged in a side area of the substantially cylindrical tampon 100. The tampon 100 is completely enclosed by the packaging material sheet 11 and is welded at the folding or winding points at its ends, so that the tampon 100 is protected against dampness and contamination from the outside.

As can be seen from Fig. 2b, after the destruction of the lines of weakness 20, 21, two sub-areas 30 of the packaging material sheet 11 can be peeled off the tampon 100 in opposite directions. Even if the tampon has expanded after being packed, the packaging material sheet 11 can be peeled off the tampon 100 without difficulty.

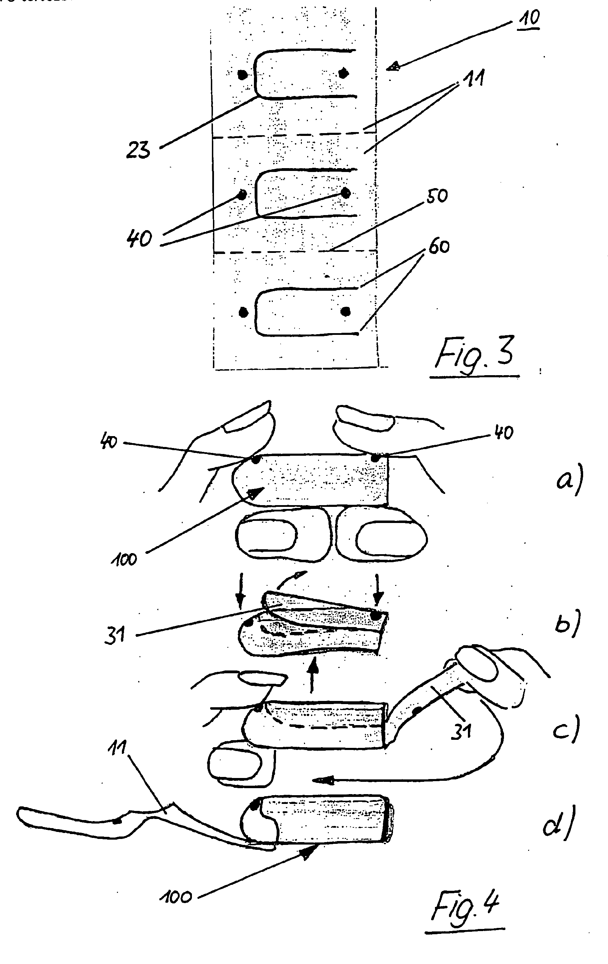

Figs 3 and 4 show a second embodiment of a packaging material sheet 11 according to the invention in a similar illustration. Corresponding elements are provided with identical reference symbols.

The line of weakness 23 in this embodiment is of U-shaped design and has two end points 60. Furthermore, orientation points 40 are arranged visibly on the packaging material sheet 11, inside and outside the U. The orientation points 40 are printed on and are used firstly for the positioning and the optical monitoring of the production machines during the production of the packaging material, in particular during the introduction, preferably performed with a laser, of the line of weakness 23, and secondly they are used by the user as an indication as to where pressure has to be exerted on the packed product in order to destroy the line of weakness, as shown in Fig. 4 and explained below.

Fig. 4 shows in schematic form how a tampon 100 for feminine hygiene packed with the packaging material sheet 11 shown in Fig. 3 is removed. As can be seen in Fig. 4a, the user takes the packed tampon in both hands and bends it downward in the outer areas in relation to the central area, in the direction of the arrows shown in Fig. 4b. The flexible tampon gives way, and the line of weakness is destroyed, so that a lateral area 31, lying at the top in Fig. 4b, folds open upward. As Figs 4c and 4d show, this area 31, corresponding to the inner area of the "U", can be gripped by the user and led around the tampon, so that the entire packaging material sheet 11 is peeled off the tampon 100 in one piece.

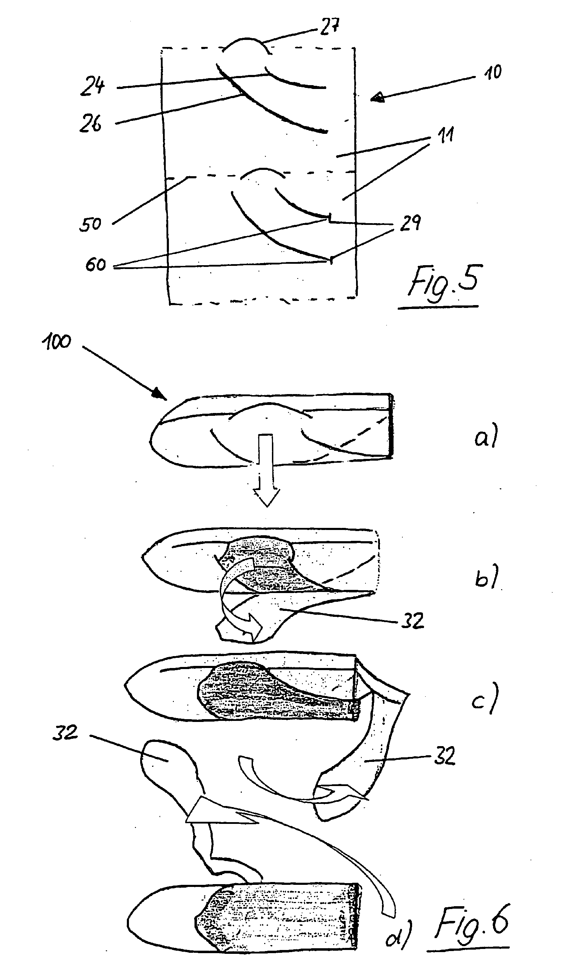

A third embodiment is depicted in Figs 5 and 6 in a way similar to the preceding figures. Here, too, corresponding elements are identified by identical reference symbols.

Fig. 5 again shows a packaging material sheet 10, from which a packaging material sheet 11 according to the invention can be separated. The packaging material sheet 11 has two slightly curved lines of weakness 24, 26. The packaging material sheet 11 is substantially rectangular but, in a side area, has a curve 27 which has the shape of a circular segment and extends over the otherwise rectangular cross section, and also a cutout corresponding to the curve 27 in its opposite side area.

In addition, at two end points 60 of the lines of weakness 24, 26, the packaging material sheet 11 in each case has a reinforcing area 29, formed as a line in this embodiment, which prevents the packaging material sheet 11 tearing beyond the lines of weakness 24, 26 when the product is being unpacked, and in this way ensures peeling off in one piece.

As opposed to the embodiments shown in Figs 1 to 4, the lines of weakness 24, 26 are not composed of individual weak points but consist of a continuous line of weakness, over whose entire extent the thickness of the material is reduced.

Fig. 6 shows schematically, in a similar way to Figs 2 and 4, the unpacking of a tampon 100 which has been packed with the packaging material sheet 11 illustrated in Fig. 5.

As shown in Figs 6a and 6b an area 32 of the packaging material sheet 11 located under the circular-segment like curve 27 in the figures is firstly pulled downward (in the figure) and then, as illustrated in Figs 6c and 6d, led around firstly in the direction of its recovery end and then around the tampon, in a similar way to the packaging material sheet 11 shown in Figs 3 and 4, so that the packaging material sheet 11 is peeled off the tampon 100 gradually and in one piece.

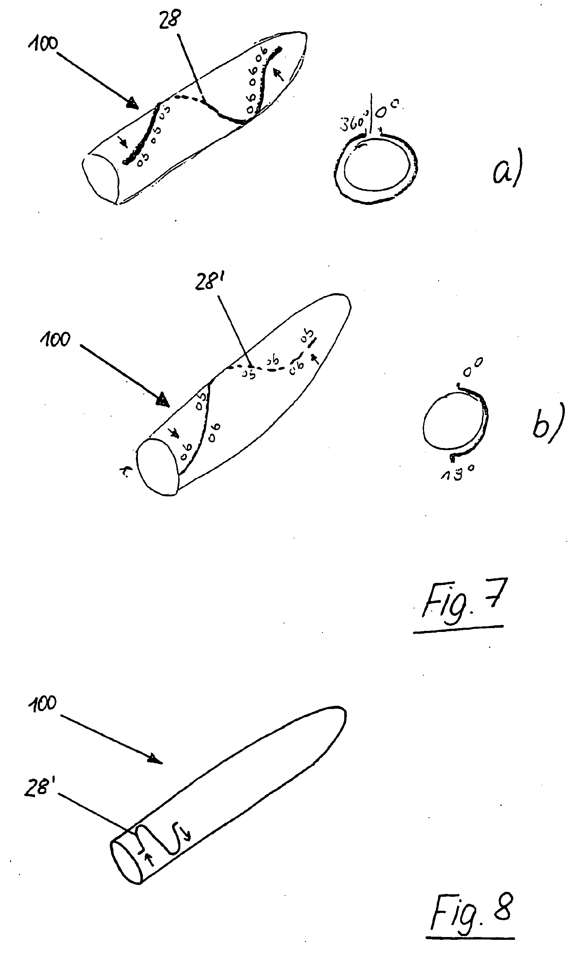

Fig. 7 shows a tampon 100 which has been packed with two versions of a fourth embodiment of the packaging material sheet 11 according to the invention. The packaging material sheet has a substantially sinusoidal line of weakness 28, 28'. In the embodiment shown in Fig. 7a, the sinusoidal line of weakness 28 extends substantially around the entire circumference of the tampon 100, while the line of weakness 28' shown in Fig. 7b extends only on one side of the tampon 100, that is to say in an angular range of 180° around the circumference of the tampon 100.

The lines of weakness 28, 28' are formed by lining up individual weak points (not illustrated) in a manner similar to that in the embodiments shown in Figs 1 to 4. The weak points are points with reduced material thickness. Instead of the reduced thickness, however, a perforation along the lines of weakness 28, 28' is also possible.

Fig. 8 shows a tampon 100 which has been packed with a fifth embodiment of the packaging material sheet 11 according to the invention and is very similar to the version shown in Fig. 7b.

This packaging material sheet also has a substantially sinusoidal line of weakness 28', but it extends only in a rear area of the tampon 100, in the vicinity of the recovery end of the tampon 100, over about 20% of the length of the tampon 100 in this embodiment. As in the version of the fourth embodiment shown in Fig. 7b, the line of weakness extends around the circumference of the tampon 100 only over an angular range of 180°, but it is also possible to provide an embodiment corresponding to the version shown in Fig. 7a, whose line of weakness extends substantially around the entire circumference of the tampon.

All the packaging sheets shown in the exemplary embodiments described above have lines of weakness with at least two end points 60 lying within the packaging sheets 11. In addition, in all the embodiments shown, the packaging material consists of a cellophane film with a thickness of about 22 µm. The weakening sections or weak points 25 and the continuous lines of weakness 24, 26 have a thickness of about 7 to 10µm. The weak points and the lines of weakness are introduced into the cellophane film by means of a laser while maintaining a constant transport speed of said cellophane film. Instead of the cellophane film, however, a polyethylene film or another plastic film can also be used.

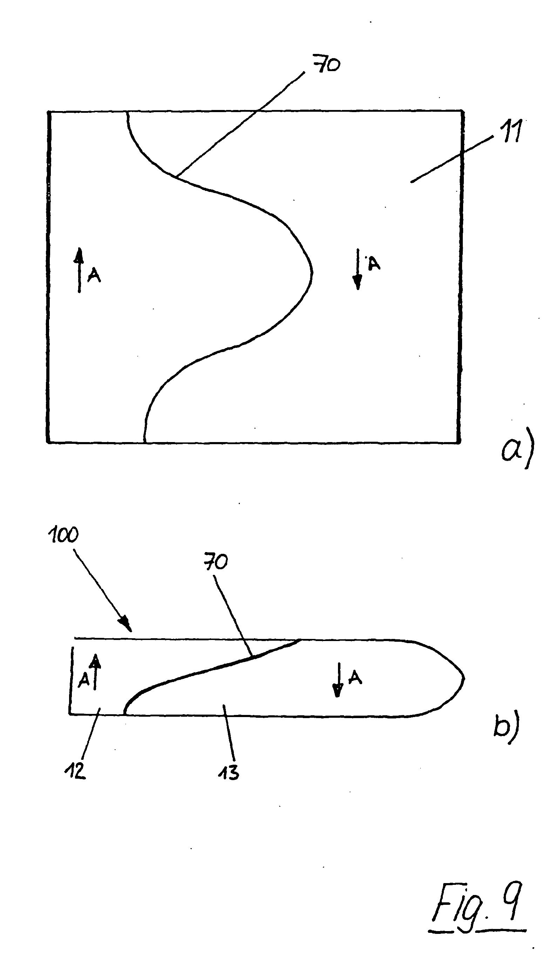

Fig. 9a shows a packaging material sheet 11 of a first embodiment according to the invention. The packaging material sheet 11 likewise consists of cellophane and has a continuous line of destruction 70, the line of destruction 70 being substantially sinusoidal and, over the width of the packaging material sheet 11, substantially representing one complete sinusoidal oscillation.

In this embodiment, the line of destruction 70 is a line of weakness formed by lining up individual weak points (not illustrated).

The weak points are points with a reduced material thickness. Instead of the reduced thickness, however, a perforation along the line of destruction 70 would also be possible in this embodiment.

Fig. 9b shows a tampon 100 which has been packed with the embodiment of the packaging material sheet 11 shown in Fig. 9a.

The line of destruction 70 extends approximately over 50% of the length of the tampon 100, so that after the destruction of the line of destruction 70, which can be brought about, for example, by rotating the packaging material sheet along the arrows A shown in Figs 9a and 9b, the two sleeve-like parts 12,13 of the packaging material sheet 11 which are produced can be pulled off the tampon 100 in the longitudinal direction of the tampon and, in relative terms, in opposite directions.

Since the sleeve-like parts 12,13 of the packaging material sheet do not enclose the tampon over an angular range of 360° over a large part of their longitudinal extent, but are open at one side over a large part of the longitudinal extent, the frictional forces as the parts 12,13 are pulled off are reduced, which permits them to be pulled off considerably more easily, even in the case of a tampon 100 which has expanded slightly following production.

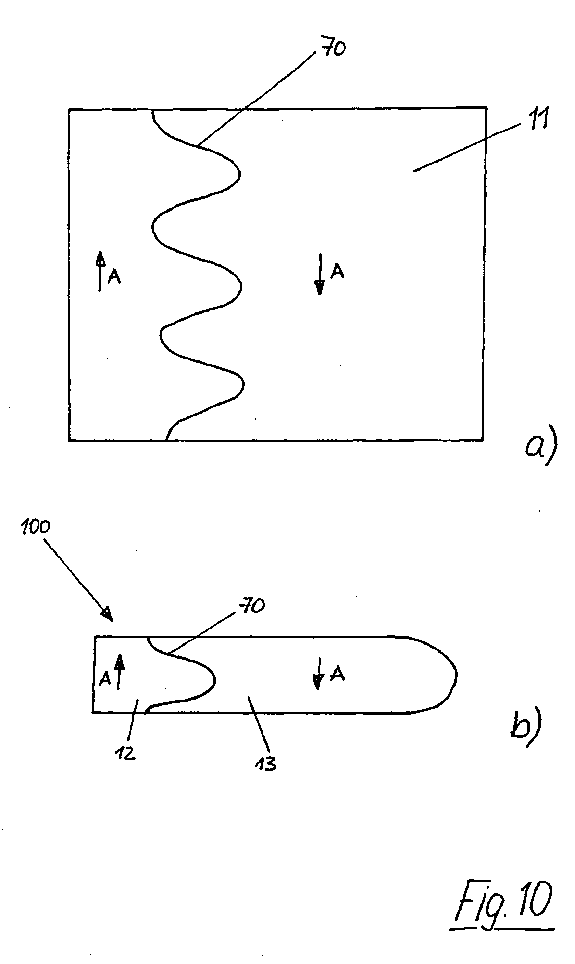

Figs 10a and 10b show a second embodiment according to the invention, which is very similar to the embodiment shown in Fig. 9. As opposed to the line of destruction 70 of the embodiment shown in Fig. 9, the line of destruction 70 in this embodiment is wavy and extends only over a range of about 20% of the longitudinal extent of the tampon 100.

The effects and mode of action of the packaging material sheet according to the invention correspond to the embodiment in Fig. 9.

Of course, it is also possible to implement different geometric embodiments. In particular, it is also possible to provide a line of destruction which is composed of a plurality of rectilinear sections, so that the line of destruction 70 has corners or bends at the points of contact between the rectilinear segments.

The features presented in the claims, the description and the drawings may be essential to the invention both individually and in any combination.

### List of reference numerals

- 10: Packaging material web
- 11: Packaging material sheet
- 12: Part (packaging material sheet 11)
- 13: Part (packaging material sheet 11)
- 20: Line of weakness
- 21: Line of weakness
- 23: Line of weakness (U-shaped)
- 24: Line of weakness (curved)
- 25: Weak points
- 26: Line of weakness (curved)
- 27: Curve (circular-segment shaped)
- 28: Line of weakness (sinusoidal)
- 28': Line of weakness (sinusoidal)
- 29: Reinforcing area
- 30: Sub-area (packaging material sheet 11)
- 31: Area
- 32: Area
- 40: Orientation points
- 50: Line of weakness between packaging material sheets 11
- 60: End point (line of weakness)
- 70: Line of destruction
- 100: Tampon

## Claims

1. A tampon wrapped in a packaging material sheet having at least one line of weakness to form a packaged product, **characterized in that** the at least one line of weakness extends over at least a part of the longitudinally extension of the packaged product, whereas only an at least partial destruction of the at least one line of weakness (20, 21, 23, 24, 25, 26, 28, 28') makes a portion (30, 32) of the packaging material available to be gripped by a user in order to peel off the packaging material sheet from the tampon.

2. The tampon as claimed in claim 1, wherein said packaging material can be peeled off the product in one piece.

3. The tampon as claimed in claim 1 or 2, wherein the at least one line of weakness (20, 21, 23, 24, 26, 28, 28') has at least one end point (60) within the packaging material sheet.

4. The tampon as claimed in claim 1 or 2, wherein the at least one line of weakness (20, 21, 23, 24, 26, 28, 28') has at least two end points (60) within the packaging material sheet.

5. The tampon as claimed in one of claims, 1 to 4, wherein the at least one line of weakness (20, 21, 23, 24, 26, 28, 28') can be destroyed by compressing and/or bending the packed product (100).

6. The tampon as claimed in one of claims 1 to 4, wherein the at least one line of weakness (20, 21, 23, 24, 26, 28, 28') can be destroyed by the at least partially opposite action of force on the packaging material sheet (11).

7. The tampon as claimed in one of the preceding claims, wherein the at least one line of Weakness (20, 21, 23, 24, 26, 28, 28') is curved and/or runs at least partially in at least two mutually different directions.

8. The tampon as claimed in one of the preceding claims, wherein a line of weakness (23) runs in a U-shape.

9. The tampon as claimed in one of claims 1 to 7, wherein a line of weakness (28, 28') has a sinusoidal course.

10. The tampon as claimed in one of claims 1 to 7, wherein two lines of weakness (24 ,26) arranged at a distance from each other run in curves.

11. The tampon as claimed in one of claims 1 to 7, wherein there are two mutually crossing lines of weakness (20, 21).

12. The tampon as claimed in claim 11, wherein the two lines of weakness (20, 21) run substantially rectilinearly.

13. The tampon as claimed in one of the preceding claims, wherein the at least one line of weakness (20, 21, 23, 24, 26, 28') is formed from weakening sections or weak points (25) arranged at a distance from one another.

14. The tampon as claimed in one of the preceding claims, wherein the at least one line of weakness (20, 21, 23, 24, 26, 28, 28') and/or the weakening sections or weak points (25) have different and/or varying dimensions.

15. The tampon as claimed in one of the preceding claims, wherein the at least one line of weakness (20, 21, 23, 24, 26, 28, 28') is formed by a reduced material thickness of the packaging material sheet (11) and/or by perforating the packaging material sheet.

16. The tampon as claimed in one of the preceding claims, wherein the line of weakness extends over at least 20% of the longitudinal extent of the pack.

17. The tampon as claimed in one of the preceding claims, wherein the line of weakness extends over at least 50% of the longitudinal extent of the pack.

18. The tampon as claimed in one of the preceding claims, wherein the line of weakness extends over at least 70% of the longitudinal extent of the pack.

## Patentansprüche

1. Tampon, umhüllt von einer Verpackungsmaterialbahn mit wenigstens einer Schwächungslinie, um ein verpacktes Produkt auszubilden, **dadurch gekennzeichnet, dass** sich die wenigstens eine Schwächungslinie über wenigstens einen Teil der longitudinalen Ausdehnung des verpackten Produkts erstreckt, wobei nur eine wenigstens teilweise Zerstörung der wenigstens einen Schwächungslinie (20, 21, 23, 24, 25, 26, 28, 28') einen Abschnitt (30, 32) des Verpackungsmaterials verfügbar macht, um von einem Nutzer ergriffen zu werden, um die Verpackungsmaterialbahn vom Tampon abzuziehen.

2. Tampon, wie in Anspruch 1 beansprucht, **dadurch gekennzeichnet, dass** das Verpackungsmaterial vom Produkt in einem Stück abgezogen werden kann.

3. Tampon, wie in Anspruch 1 oder 2 beansprucht, **dadurch gekennzeichnet, dass** die wenigstens eine Schwächungslinie (20, 21, 23, 24, 26, 28, 28') wenigstens einen Endpunkt (60) innerhalb der Verpackungsmaterialbahn aufweist.

4. Tampon, wie in Anspruch 1 oder 2 beansprucht, **dadurch gekennzeichnet, dass** die wenigstens eine Schwächungslinie (20, 21, 23, 24, 26, 28, 28') wenigstens zwei Endpunkte (60) innerhalb der Verpackungsmaterialbahn aufweist.

5. Tampon, wie in einem der Ansprüche 1 bis 4 beansprucht, **dadurch gekennzeichnet, dass** die wenigstens eine Schwächungslinie (20, 21, 23, 24, 26, 28, 28') zerstört werden kann, indem das verpackte Produkt (100) zusammengedrückt und/oder gebogen wird.

6. Tampon, wie in einem der Ansprüche 1 bis 4 beansprucht, **dadurch gekennzeichnet, dass** die wenigstens eine Schwächungslinie (20, 21, 23, 24, 26, 28, 28') durch die wenigstens zum Teil entgegengesetzte Einwirkung von Kraft auf die Verpackungsmaterialbahn (11) zerstört werden kann.

7. Tampon, wie in einem der vorangehenden Ansprüche beansprucht, **dadurch gekennzeichnet, dass** die wenigstens eine Schwächungslinie (20, 21, 23, 24, 26, 28, 28') gekrümmt ist und/oder wenigstens zum Teil in wenigstens zwei gegenseitig unterschiedlichen Richtungen verläuft.

8. Tampon, wie in einem der vorangehenden Ansprüche beansprucht, **dadurch gekennzeichnet, dass** eine Schwächungslinie (23) in einer U-Form verläuft.

9. Tampon, wie in einem der Ansprüche 1 bis 7 beansprucht, **dadurch gekennzeichnet, dass** eine Schwächungslinie (28, 28') einen sinusförmigen Verlauf hat.

10. Tampon, wie in einem der Ansprüche 1 bis 7 beansprucht, **dadurch gekennzeichnet, dass** zwei Schwächungslinien (24, 26), die in einem Abstand voneinander angeordnet sind, in Kurven verlaufen.

11. Tampon, wie in einem der Ansprüche 1 bis 7 beansprucht, **dadurch gekennzeichnet, dass** es zwei sich gegenseitig kreuzende Schwächungslinien (20, 21) gibt.

12. Tampon, wie in Anspruch 11 beansprucht, **dadurch gekennzeichnet, dass** die zwei Schwächungslinien (20, 21) im Wesentlichen geradlinig verlaufen.

13. Tampon, wie in einem der vorangehenden Ansprüche beansprucht, **dadurch gekennzeichnet, dass** die wenigstens eine Schwächungslinie (20, 21, 23, 24, 26, 28') ausgebildet wird aus Schwächungsabschnitten oder Schwächungspunkten (25), die in einem Abstand von einander angeordnet sind.

14. Tampon, wie in einem der vorangehenden Ansprüche beansprucht, **dadurch gekennzeichnet, dass** die wenigstens eine Schwächungslinie (20, 21, 23, 24, 26, 28, 28') und/oder die Schwächungsabschnitte oder Schwächungspunkte (25) unterschiedliche und/oder variierende Abmaße haben.

15. Tampon, wie in einem der vorangehenden Ansprüche beansprucht, **dadurch gekennzeichnet, dass** die wenigstens eine Schwächungslinie (20, 21, 23, 24, 26, 28, 28') ausgebildet wird durch eine reduzierte Materialdicke der Verpackungsmaterialbahn (11) und/oder durch das Perforieren der Verpackungsmaterialbahn.

16. Tampon, wie in einem der vorangehenden Ansprüche beansprucht, **dadurch gekennzeichnet, dass** sich die Schwächungslinie über wenigstens 20 % der longitudinalen Ausdehnung der Packung erstreckt.

17. Tampon, wie in einem der vorangehenden Ansprüche beansprucht, **dadurch gekennzeichnet, dass** sich die Schwächungslinie über wenigstens 50 % der longitudinalen Ausdehnung der Packung erstreckt.

18. Tampon, wie in einem der vorangehenden Ansprüche beansprucht, **dadurch gekennzeichnet, dass** sich die Schwächungslinie über wenigstens 70 % der longitudinalen Ausdehnung der Packung erstreckt.

## Revendications

1. Tampon enveloppé dans une feuille de matériau d'emballage ayant au moins une ligne de faiblesse afin de former un produit emballé, **caractérisé en ce que** la au moins une ligne de faiblesse s'étend sur au moins une partie de l'extension longitudinale du produit emballé, tandis que seulement une destruction au moins partielle de la au moins une ligne de faiblesse (20, 21, 23, 24, 25, 26, 28, 28') rend une partie (30, 32) du matériau d'emballage disponible pour être saisie par un utilisateur afin d'ôter la feuille de matériau d'emballage du tampon.

2. Tampon selon la revendication 1, dans lequel ledit matériau d'emballage peut être ôté du produit en une seule pièce.

3. Tampon selon la revendication 1 ou 2, dans lequel la au moins une ligne de faiblesse (20, 21, 23, 24, 26, 28, 28') possède au moins un point d'extrémité (60) dans la feuille de matériau d'emballage.

4. Tampon selon la revendication 1 ou 2, dans lequel la au moins une ligne de faiblesse (20, 21, 23, 24, 26, 28, 28') a au moins deux points d'extrémité (60) dans la feuille de matériau d'emballage.

5. Tampon selon l'une quelconque des revendications 1 à 4, dans lequel la au moins une ligne de faiblesse (20, 21, 23, 24, 26, 28, 28') peut être détruite en comprimant et/ou pliant le produit emballé (100).

6. Tampon selon l'une quelconque des revendications 1 à 4, dans lequel la au moins une ligne de faiblesse (20, 21, 23, 24, 26, 28, 28') peut être détruite par l'action au moins partiellement opposée d'une force sur la feuille de matériau d'emballage (11).

7. Tampon selon l'une quelconque des revendications précédentes, dans lequel la au moins une ligne de faiblesse (20, 21, 23, 24, 26, 28, 28') est incurvée et/ou s'étend au moins pour partie dans au moins deux directions mutuellement différentes.

8. Tampon selon l'une quelconque des revendications précédentes, dans lequel une ligne de faiblesse (23) s'étend en forme de U.

9. Tampon selon l'une quelconque des revendications 1 à 7, dans lequel une ligne de faiblesse (28, 28') suit un trajet sinusoïdal.

10. Tampon selon l'une quelconque des revendications 1 à 7, dans lequel deux lignes de faiblesse (24, 26) agencées à une certaine distance l'une de l'autre s'étendent de manière curviligne.

11. Tampon selon l'une quelconque des revendications 1 à 7, comprenant deux lignes de faiblesse se croisant mutuellement (20, 21).

12. Tampon selon la revendication 11, dans lequel les deux lignes de faiblesse (20, 21) s'étendent de manière sensiblement rectiligne.

13. Tampon selon l'une quelconque des revendications précédentes, dans lequel la au moins une ligne de faiblesse (20, 21, 23, 24, 26, 28") est formée de tronçons affaiblis ou de points faibles (25) agencés à une certaine distance les uns des autres.

14. Tampon selon l'une quelconque des revendications précédentes, dans lequel la au moins une ligne de faiblesse (20, 21, 23, 24, 26, 28, 28') et/ou les tronçons affaiblis ou les points faibles (25) ont des dimensions différentes et/ou variables.

15. Tampon selon l'une quelconque des revendications précédentes, dans lequel la au moins une ligne de faiblesse (20, 21, 23, 24, 26, 28, 28') est constituée d'une épaisseur de matériau réduite de la feuille de matériau d'emballage (11) et/ou par perforation de la feuille de matériau d'emballage.

16. Tampon selon l'une quelconque des revendications précédentes, dans lequel la ligne de faiblesse s'étend sur au moins 20 % de l'étendue longitudinale de l'emballage.

17. Tampon selon l'une quelconque des revendications précédentes, dans lequel la ligne de faiblesse s'étend sur au moins 50 % de l'étendue longitudinale de l'emballage.

18. Tampon selon l'une quelconque des revendications précédentes, dans lequel la ligne de faiblesse s'étend sur au moins 70 % de l'étendue longitudinale de l'emballage.
